(19) Europäisches Patentamt
European Patent Office
Office européen des brevets

(11) Publication number: 0 320 954 A2

# (12) EUROPEAN PATENT APPLICATION

(21) Application number: 88121042.1

(22) Date of filing: 15.12.88

(51) Int. Cl.[4]: C08F 12/32 , C08F 20/10 , C08F 18/00 , G03C 1/04 , C09D 3/74 , C07D 327/08

(30) Priority: 15.12.87 JP 315267/87
29.11.88 JP 301541/88

(43) Date of publication of application:
21.06.89 Bulletin 89/25

(84) Designated Contracting States:
DE FR GB IT

(71) Applicant: NIPPON OIL AND FATS COMPANY, LIMITED
10-1, Yuraku-cho 1-chome
Chiyoda-ku Tokyo100(JP)

(72) Inventor: Ishidoya, Masahiro
3654-2, Maiokacho Totsuka-ku
Yokohama-shi Kanagawa-ken(JP)
Inventor: Nakamichi, Toshihiko
2-1-912, Higashi Kugenuma
Fujisawa-shi Kanagawa-ken(JP)

(74) Representative: Wehnert, Werner, Dipl.-Ing. et al
Patentanwälte Dipl.-Ing. Graalfs, Dipl.-Ing. Hauck, Dipl.-Ing. Wehnert, Dr.-Ing. Döring
Mozartstrasse 23
D-8000 München 2(DE)

## (54) Transparent resin and transparent resin products.

(57) A transparent resin is obtained by polymerizing a feed starting material containing at least one monomer represented by the formula (I)

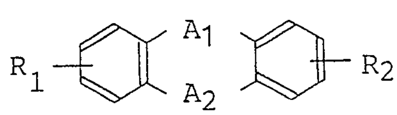
(I)

wherein $R_1$- represents $-CH=CH_2$, $CH_2-CH=CH_2$, $-COOC(CH_3)=CH_2$, $-COOCH_2-CH=CH_2$, $-OCOO-CH_2-CH=CH_2$, $-OCOO-CH_2-C(CH_3)=CH_2$, $-OCOCH=CH_2$ or $-OCOC(CH_3)=CH_2$, $R_2$- represents -H, $-OCH_3$, $-SCH_3$, $-CH_3$, $-C_2H_5$ or $-CH=CH_2$, and $A_1$ and $A_2$ each represent O or S. A transparent optical article is constituted of the transparent resin obtained by polymerizing the monomer of the formula (I). A paint composition comprises the transparent resin obtained by polymerizing the monomer of the formula (I).

EP 0 320 954 A2

FIG. 1

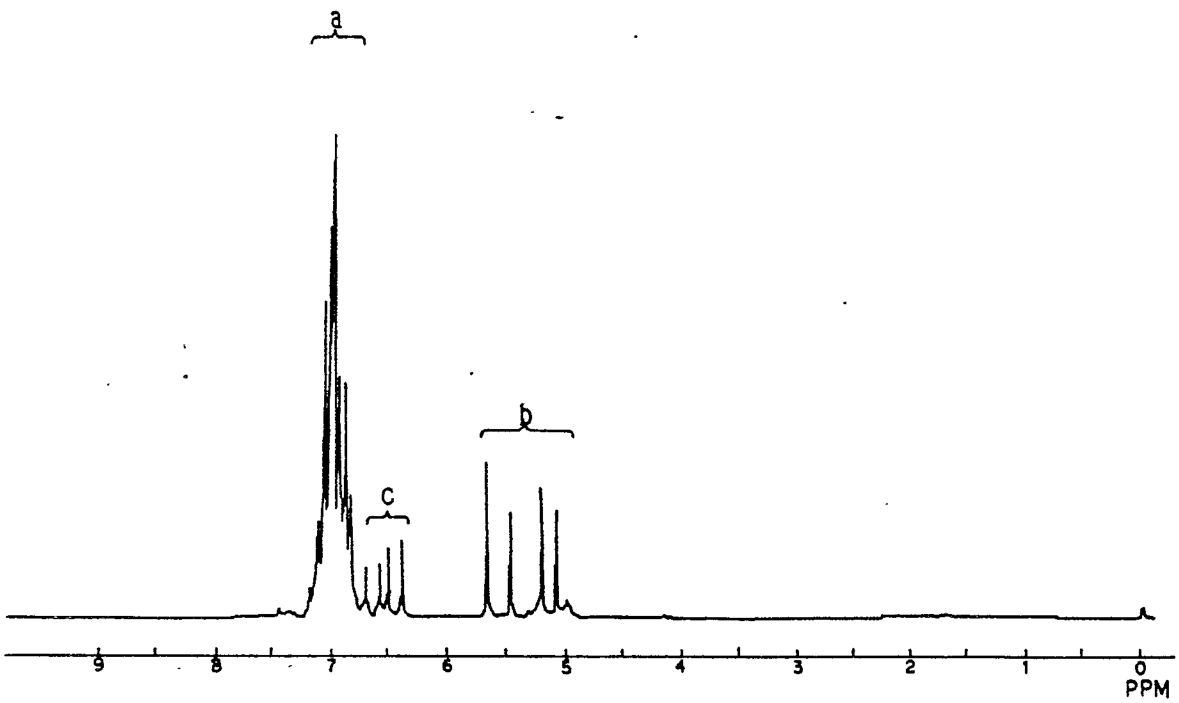

## Transparent Resin and Transparent Resin Products

BACKGROUND OF THE INVENTION:

This invention relates to a transparent resin, a high refractive index transparent optical article, a paint composition and a monomer. More particularly, it relates to a transparent resin utilizable as an optical material, a molding material, an optical fiber a film or paint, a lightweight transparent optical article having a high refractive index, a paint composition having a high refractive index and excellent transparency, and a novel monomer utilizable as the feed starting material for preparation of transparent resin products by polymerization.

In recent years, an organic material is attracting attention as an optical material in place of the conventional inorganic material and the practical utilization of such organic materials are rapidly undergoing. This is because the organic optical material is superior in lightness of weight, shock resistance and machinability to the inorganic material. Above all, by reason of safety , the organic optical material is preferentially used as lenses for eyeglasses to take the place of the inorganic optical materials. Among the organic optical materials now in current use are mainly polymethyl methacrylate and polymers of diethyleneglycolbisallyl carbonate or copolymers thereof with polyfunctional monomers.

However, the organic optical material has a refractive index as low as about 1.5, so that, when the material is used as lenses for correcting visual power, the lens thickness need be increased as compared to that of the inorganic glass lens to obtain the same diopter. This means that the needs among the consumers for lightness of weight and reduced lens thickness cannot necessarily be satisfied.

Hence, there is a strong demand for development of an organic optical material which is lightweight and transparent and which has a high refractive index.

In the field of paints, especially the paints for automotive vehicles, researches have been made to control the fluidity of the paint in dried and cured state and to reduce the contraction of the coating film. However, attempts have not been made sufficiently to elevate the refractive index of the resin itself contained in the paint composition or to improve the glossiness of the coating film. For example, the refractive indices of homopolymers of methyl methacrylate, butyl methacrylate, hexyl methacrylate, ethyl acrylate or butyl acrylate monomers, commonly used as acrylic resins for paints, amount to 1:490. 1.483, 1.481, 1.469 or 1.466, respectively, while those of homopolymers of phenyl methacrylate or styrene, known as having high refractive indices, amount to 1.571 or 1.592 respectively. Thus, in the current state of art, none of the resins employed in the paint composition has a refractive index in excess of 1.600. Hence, there is a strong demand for development of a transparent resin that has a high refractive index and that may be used as the paint composition.

It is known in general that the relation between the refractive index and the chemical composition of an organic compound may be represented by the Lorenz-Lorenz formula ("High Polymer" Vol. 33, March, 1984, pp266 to 273):

$$n_D = \sqrt{\{(2\psi + 1) / (1 - \psi)\}}$$

$$\psi = \frac{[R]}{V}$$

wherein $\eta_D$ stands for a refractive index [ R ] stands for molecular refraction, [ R] $=4^{\pi}/3 \bullet N_A \bullet \alpha$, $N_A$ representing an Avogadro number and $\alpha$ representing polarizability, V stands for molecular volume and V equals to $M/\rho$ where M stands for molecular weight and $\rho$ stands for density.

Hence, for elevating the refractive index of a compound, techniques are used to elevate the molecular refraction [ R ], that is, to introduce a chemical composition having a higher polarizability, or to reduce the molecular volume V, that is, to introduce atoms increasing the density.

In the light of the above, studies and researches are presently conducted with respect to several high refractive index organic optical materials, mainly in the field of plastic materials. Resins containing a large number of aromatic rings or halogen atoms such as chlorine or bromine have been developed as the former

type organic material having high molecular refaction [R], and resins containing heavy metals, such as lead, barium or lanthanum have been developed as the latter type organic optical material having a small molecular volume V.

However, among the above organic optical materials, the resins containing a large number of halogen atoms suffer from adverse weatherability because of chemical activities of the halogen atoms, while those containing a large number of aromatic rings suffer from adverse molding and polymerization properties due to increase in the melting points of the monomers. On the other hand, the resins containing heavy metals are increased in density and cannot be reduced in weight, while presenting problems in water-proof properties because the connecting points between the metal and the organic materials are highly susceptible to hydrolysis.

In addition, nothing is known about 3-vinylphenoxathine, 1-allylphenoxathine, 1-methacryloyloxyphenoxathin or 1-acryloyloxyphenoxathine as a feed starting material for preparing transparent resins.

## SUMMARY OF THE INVENTION:

It is a principal object of the present invention to provide a transparent resin that may be used as optical material, molding material, optical fiber film or paint.

It is another object of the present invention to provide an optical article light in weight and having high refractive index and excellent transparency.

It is a further object of the present invention to provide a paint composition having a high refractive index and excellent transparency.

It is still another object of the present invention to provide monomers exhibiting high polymerizability that may be used as the starting feed monomers in the preparartion of optical material, molding material, optical fiber, film or paint.

The above and other objects of the present invention will become apparent from the following description.

According to the present invention, there is provided a transparent resin obtained by polymerizing a feed starting material containing at least one monomer represented by the formula (I)

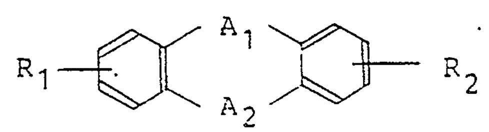

(I)

wherein $R_1$- represents $-CH=CH_2$, $-CH_2-CH=CH_2$, $-COOC(CH_3)=CH_2$, $-COOCH_2-CHCH_2$, $-OCOO-CH_2-CH=CH_2$, $-OCOO-CH_2-C(CH_3)CH_2$, $-OCOCH-CH_2$ or $-OCOC(CH_3)=CH_2$, $R_2$- represents -H, $-OCH_3$, $-SCH_3$, $-CH_3$, $-C_2H_5$ or $-CH=CH_2$, and $A_1$ and $A_2$ each represent O or S.

According to the present invention, there is also provided a transparent optical article constituted of a transparent resin obtained by polymerizing a feed starting material containing at least one monomer represented by the above formula (I), whereby to have a high refractive index.

According to the present invention, there is also provided a paint composition containing a transparent resin obtained by polymerizing a feed starting material containing at least one monomer represented by the above formula (I).

According to the present invention, there is also provided a monomer represented by the following formula (II)

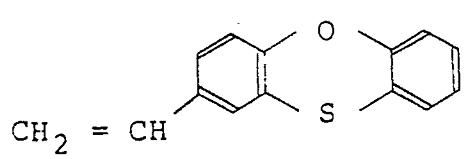

(II)

According to the present invention, there is also provided a monomer represented by the following formula (III)

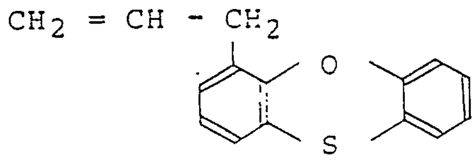

(III)

According to the present invention, there is also provided a monomer represented by the following formula (IV)

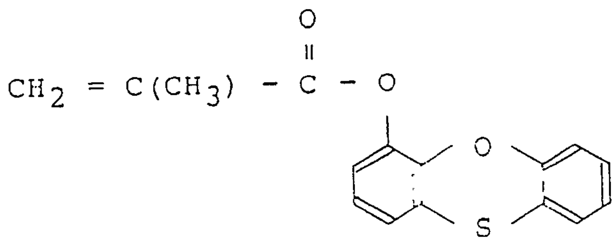

(IV)

According to the present invention, there is also provided a monomer represented by the following formula (V)

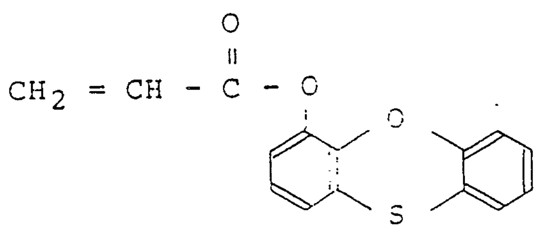

(V)

## BRIEF DESCRIPTION OF THE DRAWINGS:

Fig.1 is a chart showing a nuclear magnetic resonance (NMR) spectrum of 3-vinylphenoxathine which is a vinyl derivative obtained in the Synthesis Example 2.

Fig.2 is a chart showing an infrared (IR) spectrum of 3-vinylphenoxathine which is a vinyl derivative obtained in the Synthesis Example 2.

Fig.3 is a chart showing the NMR spectrum of 2-vinylthianthrene obtained in Synthesis Example 2.

Fig.4 is a chart showing the IR spectrum of 2-vinylthianthrene obtained in Synthesis Example 2.

Fig.5 is a chart showing the NMR spectrum of 1-allylphenoxathine obtained in Synthesis Example 3.

Fig.6 is a chart showing the IR spectrum of 1-allylphenoxathine obtained in Synthesis Example 3.

Fig.7 is a chart showing the NMR spectrum of 1-methacryloyloxyphenoxathine obtained in Synthesis Example 4.

Fig.8 is a chart showing the IR spectrum of 1-methacryloyloxyphenoxathin obtained in Synthesis Example 4.

Fig. 9 is a chart showing the NMR spectrum of 1-acryloyloxyphenoxathine obtained in Synthesis Example 4.

Fig.10 is a chart showing the IR spectrum of 1-acryloyloxyphenoxathine obtained in Synthesis Example 4.

## PREFERRED EMBODIMENTS OF THE INVENTION:

The monomer employed as the feed starting material for the transparent resins, high refracte index optical articles and paint compositions of the present invention may be represented by the following formula (I):

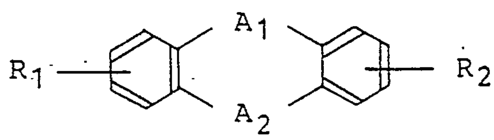

(I)

wherein $R_1$- represents $-CH=CH_2$, $-CH_2-CH=CH_2$, $-COOH(CH_3)=CH_2$, $-COOCH_2-CH=CH_2$, $-OCOO-CH_2-CHCH_2$, $-OCOO-CH_2-C(CH_3)=CH_2$, $-OCOCH=CH_2$ or $-OCOC(CH_3)=CH_2$, $R_2$ - represents -H, $-OCH_3$, $-SCH_3$, $-CH_3$, $-C_2H_5$ or $-CH=CH_2$, and $A_1$ and $A_2$ each represents O or S.

The monomers represented by the aforementioned formula (I) may preferably include for example 2-vinyldibenzo-p-dioxin, 1-allyldibenzo-p-dioxin. 1-acryloyloxydibenzo-p-dioxin, 1-methacryloyloxydibenzo-p-dioxin, 1-allyloxycarbonyldibenzo-p-dioxin, 1-methallyloxycarbonyldibenzo-p-dioxin, 1-allyloxycarbonyloxydibenzo-p-dioxin, 1-methallyloxycarbonyloxydibenzo-p-dioxin, 2-acryloyloxy-dibenzo-p-dioxin. 2-allyloxycarbonyldibenzo-p-dioxin, 1-acryloyloxyphenoxathine, 1-methacryloyloxyphenoxathine, 1-allyloxycarbonylphenoxathine, 1-methallyloxycarbonylphenoxathine, 1-allyloxycarbonyloxyphenoxathine, 1-methallyloxycarbonyloxyphenoxathine, 2-vinylthianthrene, 1-allylthianthrene, 1-acryloyloxythianthrene, 1-methacryloyloxythianthrene, 1-allyloxycarbonylthianthrene, 1-methallyloxycarbonyloxythianthrene. 2-acryloyloxythianthrene. 3-vinylphenoxathine, 1-allylphenoxathine, 1-methacryloyloxyphenoxathine or 1-acryloyloxyphenoxathine. Particularly, 3-vinylphenoxathine, 1-allylphenoxathine, 1-methacryloyloxyphenoxathine and 1-acryloyloxyphenoxathine, represented by the following formulae (II), (III), (IV) and (V)

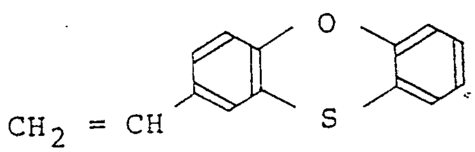

(II)

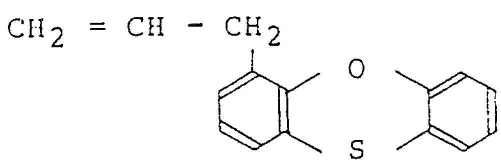

(III)

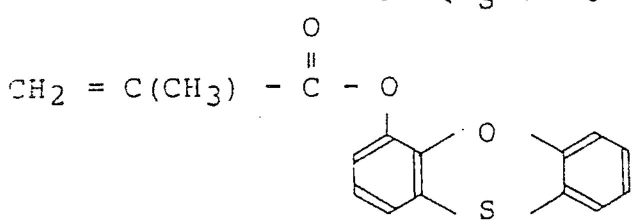

(IV)

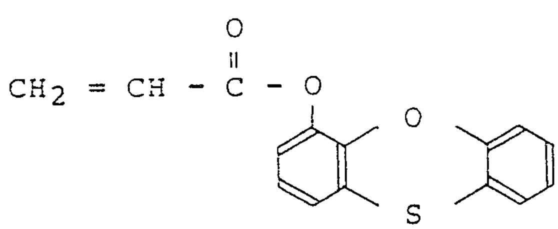

(V)

, respectively, represent novel compounds and exhibit superior properties as the feed starting monomer for the transparent resins, high refractive index optical articles and the paint compositions of the present invention. The above monomers may be used singly or in combination for polymerization.

According to the present invention, the aforementioned monomers can be synthesized easily by

combining known reactions. For example, 9,10-heterocyclic compounds, representing the basic skeleton of the aforementioned monomers, more specifically, dibenzo-p-dioxin, phenoxathine and thianthrene, may be synthesized in accordance with the following reaction fomulae:

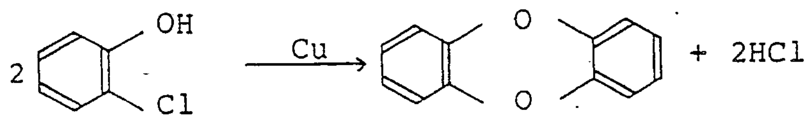


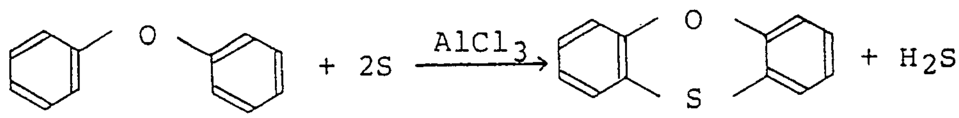


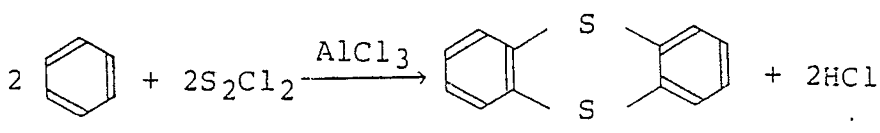


On the other hand, vinyl derivatives of the aforementioned 9,10-heterocyclic compounds may be synthesized by, for example, the following reaction formulae:

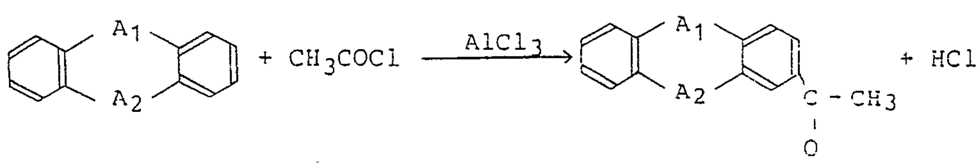


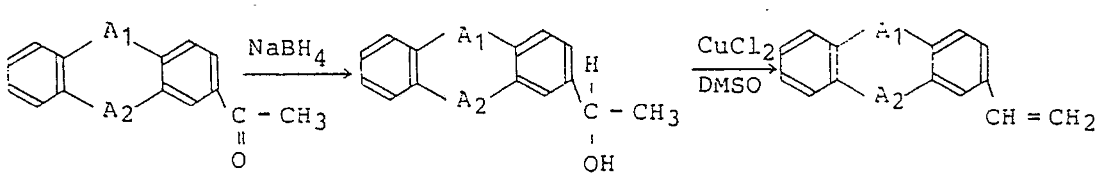


wherein $A_1$ and $A_2$ each represent O or S and DMSO represents dimethylsulfoxide.

Allyl derivatives of the 9,10-heterocyclic compounds may be produced by, for example, the following reaction formulae:

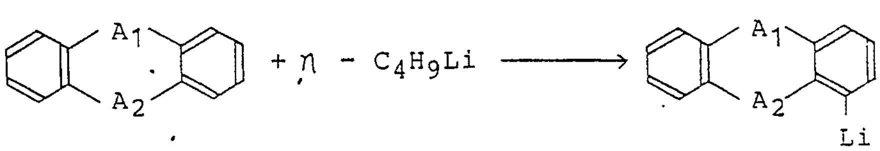

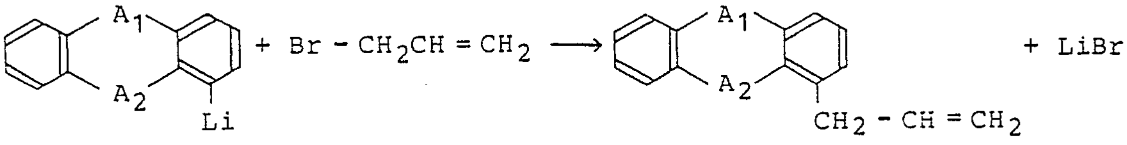

wherein $A_1$ and $A_2$ each represent O or S.

(Meth)acryloyloxy derivatives of the 9,10-heterocyclic compounds may be synthesized by, for example, the following reaction formula:

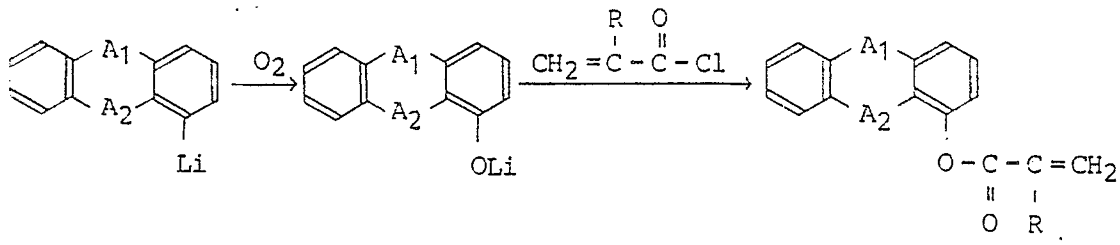


wherein $A_1$ and $A_2$ each represent O or S, and R represents a hydrogen atom or a methyl group.

Allyloxycarbonyl or allyloxycarbonyloxy derivatives of the 9,10-heterocyclic compounds may be synthesized by, for example, the following reaction formulae:

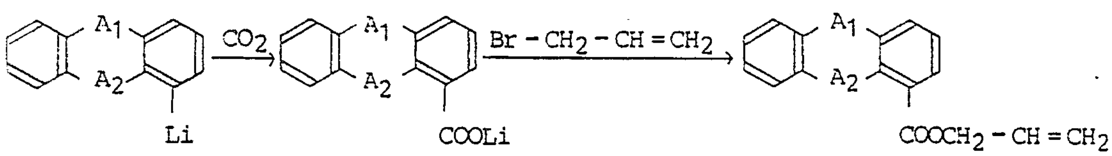


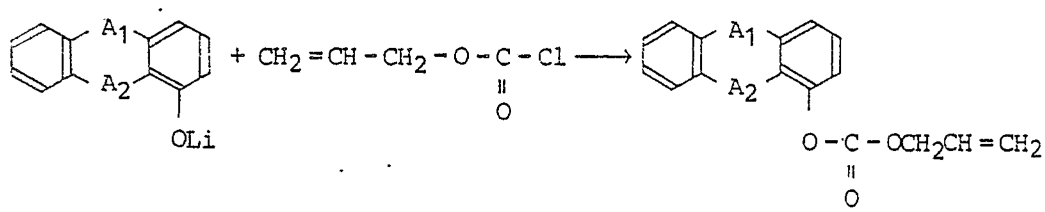

wherein $A_1$ and $A_2$ each represent O or S.

The feed starting monomer material for the transparent resins, high refractive index optical articles and paint compositions of the present invention may include, in addition to the monomers shown by the aforementioned formula (I), first comonomers copolymerizable with these monomers. There is no limitation to the first comonomers insofar as these are copolymedrizable with the aforementioned monomers and monofunctional or polyfunctional monomers may be employed as the first comonomers. The monofunctional monomers may be selected from the group consisting for example of vinyl aromatic comonomers, such as styrene, chlorostyrene, 2,4-dichlorostyrene, bromostyrene, 2,4-dibromostyrene, or vinylnaphthalene, acrylate comonomers, such as methyl acrylate, ethyl acrylate, butyl acrylate, octyl acrylate, phenyl acrylate, benzyl acrylate, chlorophenyl acrylate, dichlorophenyl acrylate trichlorophenyl acrylate, bromophenyl acrylate, dibromophenyl acrylate, tribromophenyl acrylate or β-naphthyl acrylate, methacrylate comonomers, such as methyl methacrylate, ethyl methacrylate, butyl methacrylate, isobutyl methacrylate, octyl methacrylate, cyclohexyl methacrylate, phenyl methacrylate, benzyl methacrylate, chlorophenyl methacrylate, dichlorophenyl methacrylate, trichlorophenyl methacrylate, bromophenyl methacrylate, dibromophenyl methacrylate, tribromophenyl methacrylate or β-naphthyl methacrylate, cinnamate comonomers, such as methyl cinnamate, phenyl cinnamate, chlorophenyl cinnamate, dichlorophenyl cinnamate, trichlorophenyl cinnamate, bromophenyl cinnamate, dibromophenyl cinnamate or tribromophenyl cinnamate, allyl monomers, such as allyl benzoate, 2-chloro-ally benzoate, phenyl allyl carbonate, 2-chlorophenyl allyl carbonate, 2-bromophenyl allyl carbonate, dichlorophenyl allyl carbonate, dibromophenyl allyl carbonate, trichlorophenyl allyl carbonate or tribromophenyl allyl carbonate, and mixtures thereof. Examples of the polyfunctional monomers as the aforementioned first comonomers may include aliphatic diol (meth)acrylates, such as ethyleneglycol diacrylate, ethyleneglycol dimethacrylate, diethyleneglycol diacrylate or diethyleneglycol dimethacrylate, aliphatic diol allylcarbonates, such as ethyleneglycolbisallyl carbonate or diethyleneglycolbisallyl carbonate, divinyl aromatic compounds such as divinylbenzene, diallyl esters such as diallyl phthalate, diallyl isophthlate or diallyl terephthalate or nucleus-halogenated compounds thereof, diallyl carbonates such as hydroquinone diallyl carbonate, catechol diallyl carbonate, bisphenol A diallyl carbonate, 2,2′-bis(4-hydroxyethoxyphenyl)propane diallyl carbonate, bisphenol-(S)-diallyl carbonate, bis(4-hydroxyethoxyphenyl)sufone diallyl carbonate, bisthiophenol diallyl carbonate or bis-(4-hydroxyethoxyphenyl)sulfide diallyl carbonate or nucleus-halogenated compounds thereof, diacrylates or dimethacrylates of the aforementioned aromatic polyalcohols or nucleus-halogenated compounds thereof, and mixtures thereof.

The feed starting monomers preferably employed in the transparent resins and paint compositions of the present invention may include, in addition to the aforementioned first comonomers, a second comonomer having functional groups capable of reacting with a curing agent employed in the paint compositions and copolymerizable with the aforementioned monomers. The curing agent is selected from the group consisting of polyisocyanate compounds, amino resins such as alkyletherated melamine formaldehyde resins, polyamine compounds and polycarboxylic compounds.

Examples of the aforementioned second comonomers may include hydroxyl group containing comonomers, such as 2-hydroxyethyl acrylate, 2-hydroxyethyl methacrylate, hydroxy propyl acrylate or hydroxy propyl methacrylate, epoxy group containing comonomers, such as glycidyl acrylate or glycidyl methacrylate, carboxyl group containing comonomers such as acrylic acid, methacrylic acid or maleic acid, or mixtures thereof.

The transparent resins, high refractive index optical articles or paint compositions of the present invention may be obtained by polymerizing one or more of the monomers represented by the formula (I) or by copolymerizing the aforementioned monomer and one or more of the aforementioned first and second comonomers. It is noted that the resin contents are preferably in the range of from 5 to 100 wt. % and more preferably in the range of from 20 to 100 wt. % based on the total weight of the transparent resin, high refractive index optical article or paint composition, depending on the refractive index, mechanical properties such as hardness, strength or toughness, glass transition temperatures associated with thermosoftening properties, or resistance to chemicals, such as acid- or alkali-proofness, as required for the particular usages and applications.

For polymerization or copolymerization, the well-known methods of radical polymerization or photopolymerization may be employed. The radical polymerization may be effected by, for example, block polymerization, solution polymerization or suspension polymerization. Above all, for producing a high refractive index optical article, such as lenses for eyeglasses, the cast molding method by the block polymerization method is preferred. For producing a paint composition, the solution polymerization is preferred. Examples of the radical polymerization initiators employed in the radical polymerization method may include for example peroxides such as benzoyl peroxide, p-chlorobenzoyl peroxide, 2,4-dichlorobenzoyl peroxide, tert-butylperoxypivalate, tert-butylperoxy-2-ethylhexanoate, tert-butylperoxybenzoate. acetyl peroxide, diisopropyl peroxydicarbonate or dicumyl peroxide, or azobis compounds such as azobis isobutyronitrile, azobis-2,4-dimethylvaleronitrile or dimethyl-2,2′-azobis isobutyrate, either alone or in combination. The contents of the radical polymerization initiator are preferably in the range of from 0.1 to 10 parts by weight to 100 parts by weight of the total monomers depending on the method and conditions of polymerization and the types of the comonomers. The polymerization temperature and time are usually 10° to 150° C and 1 to 100 hours, respectively, depending on the composition and reactivity of the monomers employed, and the types and amounts of the polymerization initiators. The polymerization may be effected in plural stages, if so desired.

For producing a high refractive index optical article, such as lenses for eyeglasses, by means of the aforementioned photopolymerization method, a method is preferably employed which comprises mixing 0.01 tp 10 parts by weight of a photopolymerization catalystl, such as benzophenone, benzoin or benzoinmethylether, to 100 parts by weight of a monomer or a monomer-comonomer mixture and heating the resulting mixture by irradiation from a high pressure mercury lamp or UV lamp or by a hot air drying oven so that the reaction be carried out preferably at a polymerization temperature of 5° to 80° C and for a polymerization period of 1 to 100 hours. In such a case, the radical polymerization initiators employed in the aforementioned radical polymerization method may be employed simultaneously.

In carrying out the polymerization or copolymerization, UV absorbers, such as 2-hydroxy-4-n-octyloxybenzophenone or substituted benzotriazole, anti-oxidants such as hindered phenol or hindered amine, quarternary ammonium salts or nonionic surfactants may be added to the reaction system to improve practical utilities.

The paint composition of the present invention may be employed for example as a thermoplastic resin paint dried and cured upon volatilization of solvent or as a thermosetting resin paint employed as a mixture with curing agents.

The paint composition may be employed as a clear paint composition not containing colored pigments, an enamel paint composition containing colored pigments or aluminum pigments, or as a metallic paint composition. The paint composition may also be admixed with various additives ordinarily employed in the paint compositions, namely UV absorbers, such as 2-hydroxy-4-n-octyloxybenzophenone or substituted benzotriazole, anti-oxidants or photostabilizers such as hindered phenol or hindered amine, surface conditioners such as silicone resins, curing catalysts or fluidity conditioners. These paint compositions may be produced, applied, dried and cured by employing known techniques and in accordance with the desired

usage and application.

The paint composition of the present invention may be employed for forming a coating film by the photo polymerization method by processing the monomer alone or the mixture of the monomer and the first comonomer and/or the second comonomer in the same woy as when producing the high refractive index optical article by the photopolymerization method, except that the polymerization time duration is set to 0.1 second to 30 minutes.

The transparent resin of the present invention has excellent transparency so that it may be employed as the optical material, molding material, optical fiber, film or paint. The high refractive index transparent optical article of the present invention has a high refractive index and excellent transparency so that it may be advantageously employed as the corrective eyeglass lenses. The paint composition of the present invention has a high refractive index and excellent transparency which it has been difficult to realize with the prior-art composition so that it may be used in the domain of automotive paints, paints used in domestic electrical utensils, metal paints or UV curable paints where high glossiness of the paint is desired. In addition, the monomer of the present invention exhibits high polymerizability such that it is extremely effective as the material for producing transparent resins, high refractive index optical articles or paint compositions.

## EXAMPLES OF THE INVENTION:

The present invention will be explained hereinbelow in more detail with reference to Synthesis Examples, Examples, Comparative Examples and Test Examples. However, these Examples are given only for the sake of illustration and by no means for limiting the scope of the invention.

### Synthesis Example 1

#### Synthesis of Phenoxathine

Phenoxathine was produced in accordance with the method of synthesis by C.M.Suter as disclosed in Organic Synthesis Coll., vol.2, 485, 1943. Specifically, 1886 g of biphenyl ether, 256 g of sulfur powders and 510 g of completely pulverized anhydrous aluminum chloride were charged into a 5 lit. round-bottomed flask and the reaction was carried out under sufficient agitation at 100 °C for four hours. The reaction product was then poured gradually into a beaker containing 250 ml of concentrated hydrochloric acid and 2 liters of ice to effect hydrolysis. An organic layer was separated by decantation and dried over $CaCl_2$. A fraction boiling at 116 °-125 °C/6 mmHg was obtained by reduced pressure distillation and recrystallized in methanol to produce 700 g of phenoxathine as white crystals having a melting point of 57 °C.

#### Synthesis of Thianthrene

Thianthrene was produced in accordance with the method of synthesis disclosed in the above Reference by K. Niume. Specifically, 328 g of dried benzene and 151.5 g of finely pulverized anhydrous aluminum chloride were charged into a flask of 1 liter capacity and 405 g of $S_2Cl_2$ was added dropwise for five hours under reflux. After termination of the reaction, the reaction product was poured into a beaker containing 200 ml of concentrated hydrochloric acid and 1 liter of ice to effect hydrolysis, followed by extraction with lukewarm benzene and drying over $CaCl_2$. After the solvent was removed by a rotary evaporator, the reaction product was distilled under reduced pressure to give a fraction of 190-195 °C/18 mmHg. The fraction was recrystallized in methanol to produce thianthrene as white powders having a melting point of 155 °C.

### Synthesis Example 2 Synthesis of Vinyl Derivative

1 liter of carbon disulfide, 200 g of phenoxathine synthesized in the Synthesis Example 1 and 94.2 g of acetyl chloride were charged into a flask of 2 liter capacity. Under cooling of the reaction vessel with ice and continued agitation, finely pulverized anhydrous aluminum chloride was added gradually for one hour.

The reaction was continued at 50 °C for five hours. The reaction product was then poured into a beaker containing 250 ml of concentrated hydrochloric acid and 2 liters of ice to effect hydrolysis followed by extraction with chloroform. An organic layer was washed with 5% NaOH and water and dried over $Na_2SO_4$. The resulting product was freed of the solvent and subjected to reduced pressure distillation at 175-200 °C/2 mmHg to produce 157g of 3-acetylphenoxathine having a melting point of 113 °C.

Then, a mixed solution of 48 g of 3-acetyl phenoxathine, 150ml of tetrahydrofuran and 100 ml of anhydrous methanol was added to a 500 ml solution of anhydrous methanol containing 37.8 g of dissolved $NaBH_4$ and reducing reaction was carried out for five hours at room temperature and then for 30 minutes under reflux. After termination of the reaction, the major portions of methanol and tetrahydrofuran were removed by a rotary evaporator. The resulting product was extracted with ethylether, dried over $Na_2SO_4$ and freed of solvents to produce 41 g of yellow oily 3-α-hydroxyethylphenoxathine.

Then, 48 g of the thus obtained 3-α-hydroxyethylphenoxathine, 132 g of dimethylsulfoxide and 1 g of anhydrous cuprous chloride were mixed together and subjected to dehydrating reaction at 175 °C for two hours. After termination of the reaction, water was added to the reaction system, and an organic layer was extracted with ethyl ether, dried over anhydrous magnesium sulfate and freed of solvents to produce a pale yellow liquid product. This product was purified in a silica gel column to produce 38 g of 3-vinylphenoxathine as white crystals having a melting point of 40 °C.

The nuclear magnetic resonance (NMR) spectrum and the infrared (IR) spectrum of the thus obtained 3-vinylphenoxathine are shown in Fig.1 and Fig.2, respectively. The position symbols entered in Fig.1 are as shown in the following formula.

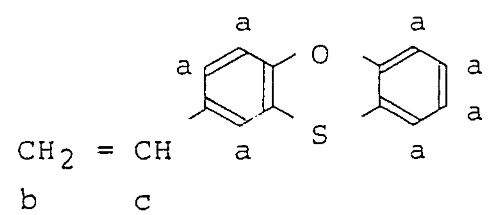

Also, an equimolar amount of thianthrene was used in place of the aforementioned phenoxathine to produce 2-vinylthianthrene as a pale yellow solid compound. The NMR spectrum and the IR spectrum of the compound are shown in Figs.3 and 4, respectively. The position symbols entered in Fig.3 are as shown in the following formula.

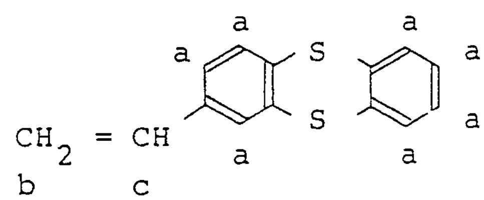

Synthesis Example 3 Synthesis of Allyl Derivative

105 ml of anhydrous ethyl ether was cooled to -5 °C and 94 ml of a 1.59M/lit. solution of n-butyllithium in hexane was added thereto. To the resulting mixture was added dropwise a 90 ml anhydrous ethyl ether solution containing 30 g of phenoxathine obtained in the Synthesis Example 1 and the resulting product was stirred for one hour at constant temperature and refluxed for two hours to produce 1-lithium phenoxathine. The product was cooled to 0 °C and there was added dropwise 18.2 g of allyl bromide. The reaction was then continued under reflux for two hours. After termination of the reaction, water was added to the reaction product to decompose unreacted butyl lithium. An organic layer was extracted with ethyl ether and dried over anhydrous sodium sulfate. The resulting product was freed of solvents by a rotary evaporator and subjected to reduced pressure distillation to produce 1-allylphenoxathine as a colorless transparent liquid compound having a boiling point of 154-160 °C/2mmHg.

The thus obtained compound had a refractive index of $n_d^{25}$ = 1.6377. Figs.5 and 6 show the NMR spectrum and the IR spectrum, respectively. The position symbols shown in Fig.5 are as shown in the

following formula.

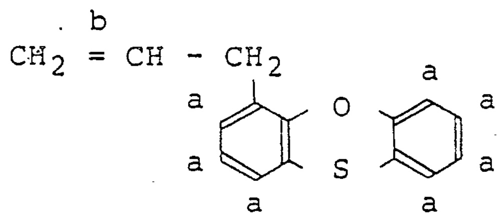

Synthesis Example 4 Synthesis of (meth)acryloyloxy Derivative

35 ml of anhydrous ether was cooled to -5 °C and 31.4 ml of a 1.59 M/lit. solution of n-butyllithium in hexane was added thereto. To the resulting solution was added dropwise a solution of 10 g of phenoxathine synthesized in Synthesis Example 1 and 30 ml of anhydrous ethyl ether for 15 minutes. After termination of the dropwise addition, the reaction was continued under reflux for two hours. After the reaction mixture was again cooled to -5 °C, two liters of dry oxygen was blown into the reaction system for 20 minutes. Then. 6.3 g of methacryloyl chloride was added dropwise for 20 minutes to the reaction system and the reaction was carried out for one hour at a constant temperature. After the temperature was reset to room temperature, to the reaction mixture was added 50 ml of water to terminate the reaction. An organic layer was extracted with ethyl ether and dried, over anhydrous sodium sulfate. The solvent was removed and the resulting product was purified and separated by silica gel chromatography to obtain 2.4 g of 1-methacryloyloxyphenoxathine. Figs.7 and 8 show the NMR spectrum and the IR spectrum. respectively. The position symbols entered in Fig.7 are as shown in the following formula.

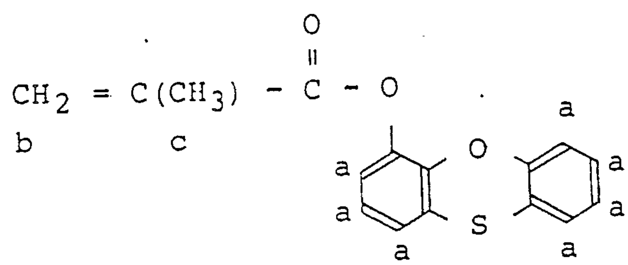

Also, using an equimolar amount of acryloyl chloride in place of methacryloyl chloride in the aformentioned Synthesis Example, 1-acryloyloxyphenoxathine was obtained. Figs.9 and 10 show the NMR spectrum and the IR spectrum, respectively. The position symbols in Fig.9 are as shown in the following formula.

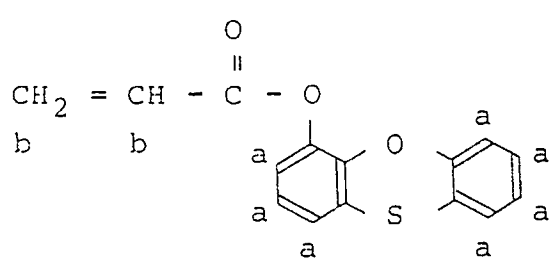

The following are abbreviations of the monomers synthesized by the above Synthesis Examples and employed in the following Examples:

3-VPT

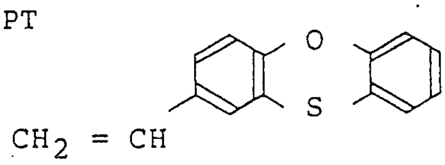

T-2VA

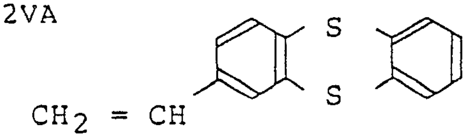

1-AlP

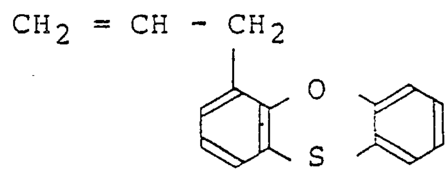

1-MAP

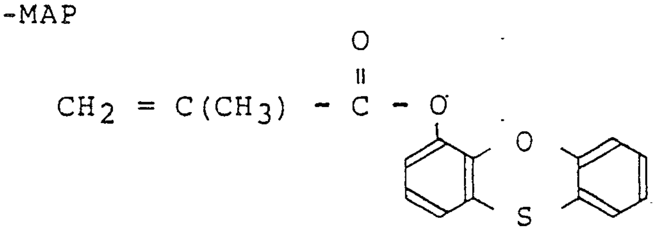

1-AcP

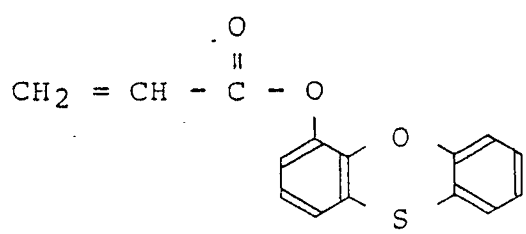

Example 1

50.0 parts by weight of 3-VPT as the monomer, 25.0 parts by weight of styrene as the monofunctional monomer, 25.0 parts by weight of divinylbenzene as the polyfunctional monomer and 2.0 parts by weight of benzoylperoxide as the polymerization initiator were mixed and dissolved together and the resulting mixture was poured into a lens molding frame made up of two spherical glasses and a gasket. Polymerization was carried out at 90° C for 15 hours in a hot air heating oven. After being cooled, the molded product was taken out of the mold frame followed by polymerization at 50° C for four hours and then at 90° C for 15 hours in the same oven to produce a transparent lens. The refractive index $\eta_D^{20}$ as measured by an Abbe refractometer was 1.649.

Examples 2 to 9

Using the same amounts of the same monomers and comonomers as shown in Table 1, and also using benzoyl peroxide as the polymerization initiator, the procedure was carried out similarly to Example 1 to produce transparent lenses.

The refractive indices of the produced lenses are as shown in Table 1.

Table 1

| Ex. | Monomer | Amount (wt.Part) | Comonomer | Amount (wt.Part) | Amount of Polymerization Initiator (wt.Part) | Refractive Index $\eta_D^{20}$ |
|---|---|---|---|---|---|---|
| 1 | 3-VPT | 50.0 | Styrene | 25.0 | 2.0 | 1.648 |
| | | | Divinyl benzene | 25.0 | | |
| 2 | 3-VPT | 50.0 | Divinyl benzene | 50.0 | 1.7 | 1.653 |
| 3 | 3-VPT | 75.0 | Divinyl benzene | 25.0 | 2.0 | 1.669 |
| 4 | 3-VPT | 100.0 | - | | 2.0 | 1.710 |
| 5 | T-2VA | 50.0 | Divinyl benzene | 50.0 | 2.0 | 1.642 |
| 6 | T-2VA | 50.0 | Styrene | 25.0 | 2.0 | 1.658 |
| | | | Divinyl benzene | 25.0 | | |
| 7 | T-2VA | 70.0 | Divinyl benzene | 30.0 | 2.5 | 1.677 |
| 8 | I-AcP | 75.0 | Divinyl benzene | 25.0 | 2.0 | 1.634 |
| 9 | I-MAP | 75.0 | Divinyl benzene | 25.0 | 2.0 | 1.615 |

Example 10

10 g of 1-AIP as the monomer was dissolved in 20 ml of dry benzene. To the resulting mixture were added 4 mmol of tetrachlorotitanium and 5 mmol of triethylaluminum chloride as the polymerization catalysts and the reaction was carried out at 23° C for 96 hours. The product was washed in a 10% HCl in methanol solution and dried under reduced pressure to obtain a transparent resin, which was worked into a film. The refractive index $\eta_D^{20}$ of the film was measured and found to be 1.654.

Example 11

64.1 parts by weight of benzene was charged into a round-bottomed flask equipped with an agitator, a dropping funnel, a thermometer and a nitrogen inlet pipe, and allowed to reflux at 80° C under a nitrogen stream. A solution mixture containing 5 parts by weight of benzene, 7.5 parts by weight of 3-VPT, 6.96 parts by weight of 2-ethylhydroxy methacrylate , 0.18 part by weight of acrylic acid, 3.76 parts by weight of methyl methacrylate, 11.60 parts by weight of butyl methacrylate and 0.90 part by weight of azobis isobutylonitrile were added dropwise for two hours from the dropping funnel, and the reaction was carried out for one hour at a constant temperature to produce a solution of a paint composition containing 30 wt. % of solids. The acid and hydroxyl values of the produced composition were 4.7 KOH mg/g and 100 KOH mg/g, respectively.

Examples 12 - 14

The operation was carried out similarly to Example 11 except changing the types and the amounts of the monomers and the comonomers of the Example 11 as shown in Table 2 to produce paint compositions.

The acid and hydroxyl values of the produced compositions are as shown in Table 2.

Comparative Examples 1 and 2

The operation was carried out similarly to Example 11 except changing the types and the amounts of the monomers and the comonomers of the Example 11 as shown in Table 2 to produce conventional paint compositions not containing the monomers of the formula (I) of the present invention. The acid values and hydroxyl values of the produced compositions are as shown in Table 2.

Table 2

| | | Ex. 12 | Ex. 13 | Ex. 14 | Comp. Ex. 1 | Comp. Ex. 2 |
|---|---|---|---|---|---|---|
| Monomer | 3-VPT | 15.00 | - | - | - | - |
| | T-2VA | - | 7.50 | - | - | - |
| | 1-MAP | - | - | 7.50 | - | - |
| Comonomer | 2-Ethylhydroxy methacrylate | 6.96 | 6.96 | 6.96 | 6.96 | 6.96 |
| | Acrylic acid | 0.18 | 0.18 | 0.18 | 0.18 | 0.18 |
| | Methyl methacrylate | - | 3.76 | 3.76 | 11.26 | 3.76 |
| | Butyl methacrylate | 7.86 | 11.60 | 11.60 | 11.60 | - |
| | Styrene | - | - | - | - | 7.50 |
| Solid Content (wt.%) | | 30.00 | 30.00 | 30.00 | 30.00 | 30.00 |
| Acid Value (KOHmg/g) | | 4.7 | 4.7 | 4.7 | 4.7 | 4.7 |
| Hydroxyl Value (KOHmg/g) | | 100 | 100 | 100 | 100 | 100 |

Test Example 1

To 82.4 parts by weight of the solution of the paint composition produced in Example 11 were added 17.6 parts by weight of "UBAN #220", a commercial name of a product manufactured by Mitsui Toatsu Kagaku Co., Ltd. (solid contents, 60 wt.%), as a cross-linking agent, to produce a paint composition. The paint composition was diluted with xylene to a viscosity as measured with Ford cup #4 of 20 seconds and spray-coated on a glass plate previously coated with a black colored paint to have a dry film thickness of 30 microns, followed by being baked for 30 minutes at 140° C.

The glossiness of the produced coated plate having a black coating and a clear coating was measured with a Dori-gon glossmeter "D-47R-6F', a trade nameof a product by Hanter Associate Laboratory, Inc.. As a result, a higher Rs value of 98.0 and a higher D/I value of 98.9 were obtained. It is noted that the Rs value stands for a value of 30° glossiness and the D/I value stands for sharpness of the coating. The higher Rs and D/I values indicate higher sharpness.

Test Examples 2-6

The operational procedure and the compositions were followed similarly to Test Example 1, except that the resin of Example 11 employed in Test Example 1 was replaced by the resins shown in Table 3, to produce a painted or coated plate. The glossiness values of the produced coated plates, as measured by the Dori-gon glossmeter, are shown in Table 3.

Table 3

| Experiment No. | 2 | 3 | 4 | 5 | 6 |
|---|---|---|---|---|---|
| Paint Composition Used | Ex. 12 | Ex. 13 | Ex. 14 | Comp. Ex. 1 | Comp. Ex. 2 |
| Rs Value | 98.0 | 98.7 | 98.4 | 83.0 | 85.2 |
| D/I Value | 98.6 | 98.5 | 98.4 | 98.0 | 98.2 |

In the Test Examples 1 to 4, higher values of sharpness and above all the higher Rs values were obtained since the paint compositions of the present invention are contained as the essential constituents. In the Test Examples 5 and 6, employing the conventional paint conditions, the produced coated plates showed only poor sharpness as compared to the coated plates employing the inventive paint compositions.

## Claims

1. A transparent resin obtained by polymerizing a feed starting material containing at least one monomer represented by the formula (I)

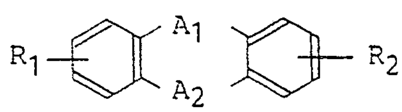

(I)

wherein $R_1$- represents $-CH=CH_2$, $-CH_2-CH=CH_2$, $-COOC(CH_3)=CH_2$, $-COOCH_2-CH=CH_2$, $-OCOO-CH_2-CH=CH_2$, $-OCOO-CH_2-C(CH_3)=CH_2$, $-OCOCH=CH_2$ or $-OCOC(CH_3)=CH_2$, $R_2$- represents -H, $-OCH_3$, $-SCH_3$, $-CH_3$, $-C_2H_5$ or $-CH=CH_2$, and $A_1$ and $A_2$ each represent O or S.

2. The transparent resin according to claim 1 wherein said monomer is selected from the group consisting of 2-vinyldibenzo-p-dioxin, 1-allyldibenzo-p-dioxin, 1-acryloyloxydibenzo-p-dioxin, 1-methacryloyloxydibenzo-p-dioxin, 1-allyloxycarbonyldibenzo-p-dioxin, 1-methallyloxycarbonyldibenzo-p-dioxin, 1-allyloxycarbonyloxydibenzo-p-dioxin, 1-methallyloxycarbonyloxydibenzo-p-dioxin, 2-acryloyloxydibenzo-p-dioxin, 2-allyloxycarbonyldibenzo-p-dioxin, 1-acryloyloxyphenoxathine, 1-methacryloyloxyphenoxathine, 1-allyloxycarbonylphenoxathine, 1-methallyloxycarbonylphenoxathine, 1-allyloxycarbonyloxyphenoxathine, 1-methallyloxycarbonyloxyphenoxathine, 2-vinylthianthrene, 1-allylthianthrene, 1 acryloyloxythianthrene, 1-methacryloyloxythianthrene, 1-allyloxycarbonylthianthrene, 1-methallyloxycarbonyloxythianthrene, 2-acryloyloxythianthrene and 2-allyloxycarbonylthianthrene.

3. The transparent resin according to claim 1 wherein said monomer is selected from the group consisting of 3-vinylphenoxathine, 1-allylphenoxathine, 1-acryloyloxyphenoxathine and 1-methacryloyloxyphenoxathine.

4. The transparent resin according to claim 1 wherein said feed starting material further contains a first comonomer copolymerizable with said monomer.

5. The transparent resin according to claim 4 wherein said first comonomer is selected from the group consisting of a monofunctional monomer and a polyfunctional monomer.

6. The transparent resin according to claim 5 wherein said monofunctional monomer is selected from the group consisting of styrene, chlorostyrene, 2,4-dichlorostyrene, bromostyrene, 2,4-dibromostyrene, vinylnaphthalene, methyl acrylate, ethyl acrylate, butyl acrylate, octyl acrylate, phenyl acrylate, benzyl acrylate, chlorophenyl acrylate, dichlorophenyl acrylate, trichlorophenyl acrylate, bromophenyl acrylate, dibromophenyl acrylate, tribromophenyl acrylate, β-naphthyl acrylate, methyl methacrylate, ethyl methacrylate, butyl methacrylate, isobutyl methacrylate, octyl methacrylate, cyclohexyl methacrylate, phenyl methacrylate, benzyl methacrylate, chlorophenyl methacrylate, dichlorophenyl methacrylate, trichlorophenyl methacrylate, bromophenyl methacrylate, dibromophenyl methacrylate, tribromopheyl methacrylate, β-naphthyl methacrylate, methyl cinnamate, phenyl cinnamate, chlorophenyl cinnamate, dichlorophenyl cinnamate, trichlorophenyl cinnamate, bromophenyl cinnamate, dibromophenyl cinnamate,

tribromophenyl cinnamate, allyl benzoate, 2-chloro-allyl benzoate, phenyl allyl carbonate, 2-chlorophenyl allyl carbonate, 2 bromophenyl allyl carbonate, dichlorophenyl allyl carbonate, dibromophenyl allyl carbonate, trichlorophenyl allyl carbonate, tribromophenyl allyl carbonate, and mixtures thereof.

7. The transparent resin according to claim 5 wherein said polyfunctional monomer is selected from the group consisting of divinylbenzene, diallyl phthalate, diallyl isophthalate, diallyl terephthalate, hydroquinone diallyl carbonate, catechol diallyl carbonate, bisphenol A diallyl carbonate, 2,2′-bis(4-hydroxyethoxyphenyl)-propane diallyl carbonate, bisphenol-(S)-diallyl carbonate, bis(4-hydroxyethoxyphenyl)sulfone diallyl carbonate, bisthiophenol diallyl carbonate, bis(4-hydroxyethoxyphenyl)sulfide diallyl carbonate, ethyleneglycol diacrylate, ethyleneglycol dimethacrylate, diethyleneglycol diacrylate, diethyleneglycol dimethacrylate, ethyleneglycol bisallyl carbonate, diethyleneglycol bisallyl carbonate, and mixtures thereof.

8. The transparent resin according to claim 1 wherein said feed starting material further contains a second comonomer copolymerizable with said monomers and having functional groups capable of reacting with a curing agent selected from the group consisting of polyisocyanate compounds, alkyletherated melamine formaldehyde resins, polyamine compounds and polycarboxylic compounds.

9. The transparent resin according to claim 8 wherein said second comonomer is selected from the group consisting of 2-hydroxyethyl acrylate, 2-hydroxyethyl methacrylate, hydroxypropyl acrylate, hydroxypropyl methacrylate, glycidyl acrylate, glycidyl methacrylate, acrylic acid, methacrylic acid, maleic acid and mixtures thereof.

10. A transparent optical article constituted of a transparent resin obtained by polymerizing a feed starting material containing at least one monomer represented by the formula (I)

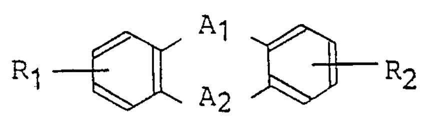

(I)

wherein $R_1$- represents $-CH=CH_2$, $-CH_2-CH=CH_2$, $-COOC(CH_3)=CH_2$, $-COOCH_2-CH=CH_2$, $-OCOO-CH_2-CH=CH_2$, $-OCOO-CH_2-C(CH_3)=CH_2$, $-OCOCH=CH_2$ or $-OCOC(CH_3)=CH_2$, $R_2$- represents -H, $-OCH_3$, $-SCH_3$, $-CH_3$, $-C_2H_5$ or $-CH=CH_2$, and $A_1$ and $A_2$ each represent O or S, whereby to have a high refractive index.

11. The transparent optical article having a high refractive index according to claim 10 wherein said monomer is selected from the group consisting of 2-vinyldibenzo-p-dioxin, 1-allyldibenzo-p-dioxin, 1-acryloyloxydibenzo-p-dioxin, 1-methacryloyloxydibenzo-p-dioxin, 1-allyloxycarbonyldibenzo-p-dioxin, 1-methallyloxycarbonyldibenzo-p-dioxin, 1-allyloxycarbonyloxydibenzo-p-dioxin, 1-methallyloxycarbonyloxydibenzo-p-dioxin, 2-acryloyloxydibenzo-p-dioxin, 2-allyloxycarbonyldibenzo-p-dioxin, 1-acryloyloxyphenoxathine, 1-methacryloyloxyphenoxathine, 1-allyloxycarbonylphenoxathine, 1-methallyloxycarbonylphenoxathine, 1-allyloxycarbonyloxyphenoxathine, 1-methallyloxycarbonyloxyphenoxathine, 2-vinylthianthrene, 1-allylthianthrene, 1-acryloyloxythianthrene, 1-methacryloyloxythianthrene, 1-allyloxycarbonylthianthrene, 1-methallyloxycarbonyloxythianthrene, 2-acryloyloxythianthrene and 2-allyloxycarbonylthianthrene.

12. The transparent optical article having a high refractive index according to claim 10 wherein said monomer is selected from the group consisting of 3-vinylphenoxathine, 1-allylphenoxathine, 1-acryloyloxyphenoxathine and 1-methacryloyloxyphenoxathine.

13. The transparent optical article having a high refractive index according to claim 10 wherein said feed starting material further contains a first comonomer copolymerizable with said monomer.

14. The transparent optical article having a high refractive index according to claim 13 wherein said first comonomer is selected from the group consisting of a monofunctional monomer and a polyfunctional monomer.

15. The transparent optical article having a high refractive index according to claim 14 wherein said monofunctional monomer is selected from the group consisting of styrene, chlorostyrene, 2,4-dichlorostyrene, bromostyrene, 2,4-dibromostyrene, vinylnaphthalene, methyl acrylate, ethyl acrylate, butyl acrylate, octyl acrylate, phenyl acrylate, benzyl acrylate, chlorophenyl acrylate, dichlorophenyl acrylate, trichlorophenyl acrylate, bromophenyl acrylate, dibromophenyl acrylate, tribromophenyl acrylate, β-naphthyl acrylate, methyl methacrylate, ethyl methacrylate, butyl methacrylate, isobutyl methacrylate, octyl methacrylate, cyclohexyl methacrylate, phenyl methacrylate, benzyl methacrylate, chlorophenyl methacrylate, dichlorophenyl methacrylate, trichlorophenyl methacrylate, bromophenyl methacrylate, dibromophenyl methacrylate, tribromopheyl methacrylate, β-naphthyl methacrylate, methyl cinnamate, phenyl cinnamate, chlorophenyl cinnamate, dichlorophenyl cinnamate, trichlorophenyl cinnamate, bromophenyl cinnamate, dibromophenyl cinnamate, tribromophenyl cinnamate, allyl benzoate, 2-chloro-allyl benzoate, phenyl allyl carbonate, 2-chlorophenyl allyl carbonate, 2-bromophenyl allyl carbonate, dichlorophenyl allyl carbonate, dibromophenyl allyl carbonate, trichlorophenyl allyl carbonate, tribromophenyl allyl carbonate, and mixtures thereof.

16. The transparent optical article having a high refractive index according to claim 14 wherein said polyfunctional monomer is selected from the group consisting of divinylbenzene, diallyl phthalate, diallyl isophthalate, diallyl terephthalate, hydroquinone diallyl carbonate, catechol diallyl carbonate, bisphenol A diallyl carbonate, 2,2′-bis(4-hydroxyethoxyphenyl)propane diallyl carbonate, bisphenol-(S)-diallyl carbonate, bis(4-hydroxyethoxyphenyl)sulfone diallyl carbonate, bisthiophenol diallyl carbonate, bis(4-hydroxyethoxyphenyl)sulfide diallyl carbonate, ethyleneglycol diacrylate, ethyleneglycol dimethacrylate, diethyleneglycol diacrylate, diethyleneglycol dimethacrylate, ethyleneglycol bisallyl carbonate, diethyleneglycol bisallyl carbonate, and mixtures thereof.

7. A paint composition comprising a transparent resin obtained by polymerizing a feed starting material containing at least one monomer represented by the formula (I)

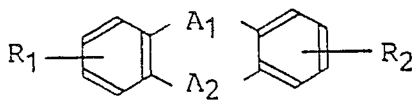

(I)

wherein $R_1$- represents $-CH=CH_2$, $CH_2-CH=CH_2$, $-COOC(CH_3)=CH_2$, $-COOCH_2-CH=CH_2$, $-OCOO-CH_2-CH=CH_2$, $-OCOO-CH_2-C(CH_3)=CH_2$, $-OCOCH=CH_2$ or $-OCOC(CH_3)=CH_2$, $R_2$- represents -H, $-OCH_3$ $-SCH_3$ $-CH_3$, $-C_2H_5$ or $-CH=CH_2$, and $A_1$ and $A_2$ each represent O or S.

18. The paint composition according to claim 17 wherein said monomer is selected from the group consisting of 2-vinyldibenzo-p-dioxin, 1-allyldibenzo-p-dioxin, 1-acryloyloxydibenzo-p-dioxin, 1-methacryloyloxydibenzo-p-dioxin, 1-allyloxycarbonyldibenzo-p-dioxin, 1-methallyloxycarbonyldibenzo-p-dioxin, 1-allyloxycarbonyloxydibenzo-p-dioxin, 1-methallyloxycarbonyloxydibenzo-p-dioxin, 2-acryloyloxydibenzo-p-dioxin, 2-allyloxycarbonyldibenzo-p-dioxin, 1-acryloyloxyphenoxathine, 1-methacryloyloxyphenoxathine, 1-allyloxycarbonylphenoxathine, 1-methallyloxycarbonylphenoxathine, 1-allyloxycarbonyloxyphenoxathine, 1-methallyloxycarbonyloxyphenoxathine, 2 vinylthianthrene, 1-allylthianthrene, 1-acryloyloxythianthrene, 1-methacryloyloxythianthrene, 1-allyloxycarbonylthianthrene, 1-methallyloxycarbonyloxythianthrene, 2-acryloyloxythianthrene and 2-allyloxycarbonylthianthrene.

19. The paint composition according to claim 17 wherein said monomer is selected from the group consisting of 3-vinylphenoxathine, 1-allylphenoxathine, 1-acryloyloxyphenoxathine and 1 methacryloyloxyphenoxathine.

20. The paint composition according to claim 17 wherein said feed starting material further contains a first comonomer copolymerizable with said monomer.

21. The paint composition according to claim 20 wherein said first comonomer is selected from the group consisting of a monofunctional monomer and a polyfunctional monomer.

22. The paint composition according to claim 21 wherein said monofunctional monomer is selected from the group consisting of styrene, chlorostyrene, 2,4-dichlorostyrene, bromostyrene, 2,4-dibromostyrene, vinylnaphthalene, methyl acrylate, ethyl acrylate, butyl acrylate, octyl acrylate, phenyl acrylate, benzyl acrylate, chlorophenyl acrylate, dichlorophenyl acrylate, trichlorophenyl acrylate, bromophenyl acrylate, dibromophenyl acrylate, tribromophenyl acrylate, β-naphthyl acrylate, methyl methacrylate, ethyl methacrylate, butyl methacrylate, isobutyl methacrylate, octyl methacrylate, cyclohexyl methacrylate, phenyl methacrylate, benzyl methacrylate, chlorophenyl methacrylate, dichlorophenyl methacrylate, trichlorophenyl methacrylate, bromophenyl methacrylate, dibromophenyl methacrylate, tribromopheyl methacrylate, β-naphthyl methacrylate, methyl cinnamate, phenyl cinnamate, chlorophenyl cinnamate, dichlorophenyl cinnamate, trichlorophenyl cinnamate, bromophenyl cinnamate, dibromophenyl cinnamate, tribromophenyl cinnamate, allyl benzoate, 2-chloro-allyl benzoate, phenyl allyl carbonate, 2-chlorophenyl allyl carbonate, 2-bromophenyl allyl carbonate, dichlorophenyl allyl carbonate, dibromophenyl allyl carbonate, trichlorophenyl allyl carbonate, tribromophenyl allyl carbonate, and mixtures thereof.

23. The paint composition according to claim 21 wherein said polyfunctional monomer is selected from the group consisting of divinylbenzene, diallyl phthalate, diallyl isophthalate, diallyl terephthalate, hydroquinone diallyl carbonate, catechol diallyl carbonate, bisphenol A diallyl carbonate, 2,2′-bis(4-hydroxyethoxyphenyl)propane diallyl carbonate, bisphenol-(S)-diallyl carbonate, bis(4-hydroxyethoxyphenyl)sulfone

diallyl carbonate, bisthiophenol diallyl carbonate, bis(4-hydroxyethoxyphenyl)sulfide diallyl carbonate, ethyleneglycol diacrylate, ethyleneglycol dimethacrylate diethyleneglycol diacrylate, diethyleneglycol dimethacrylate, ethyleneglycol bisallyl carbonate, diethyleneglycol bisallyl carbonate, and mixtures thereof.

24. The paint composition according to claim 17 wherein said feed starting material further contains a second comonomer copolymerizable with said monomers and having functional groups capable of reacting with a curing agent selected from the group consisting of polyisocyanate compounds, alkyletherated melamine formaldehyde resins, polyamine compounds and polycarboxylic compounds.

25. The paint composition according to claim 24 wherein said second comonomer is selected from the group consisting of 2-hydroxyethyl acrylate, 2-hydroxyethyl methacrylate, hydroxypropyl acrylate, hydroxypropyl methacrylate, glycidyl acrylate, glycidyl methacrylate, acrylic acid, methacrylic acid, maleic acid and mixtures thereof.

26. A monomer represented by the formula (II).

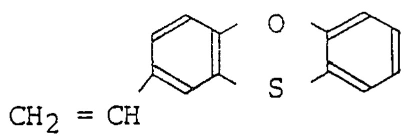

(II)

27. A monomer represented by the formula (III).

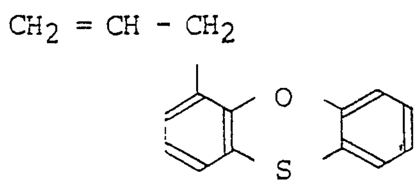

(III)

28. A monomer represented by the formula (IV).

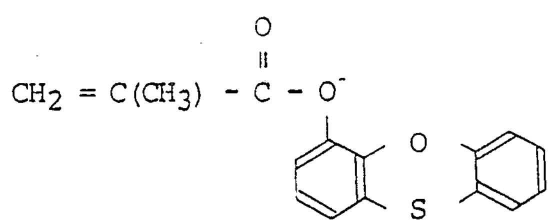

(IV)

29. A monomer represented by the formula (V).

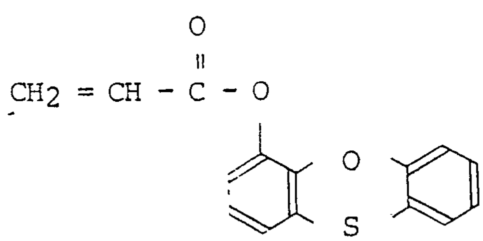

(V)

FIG. 1

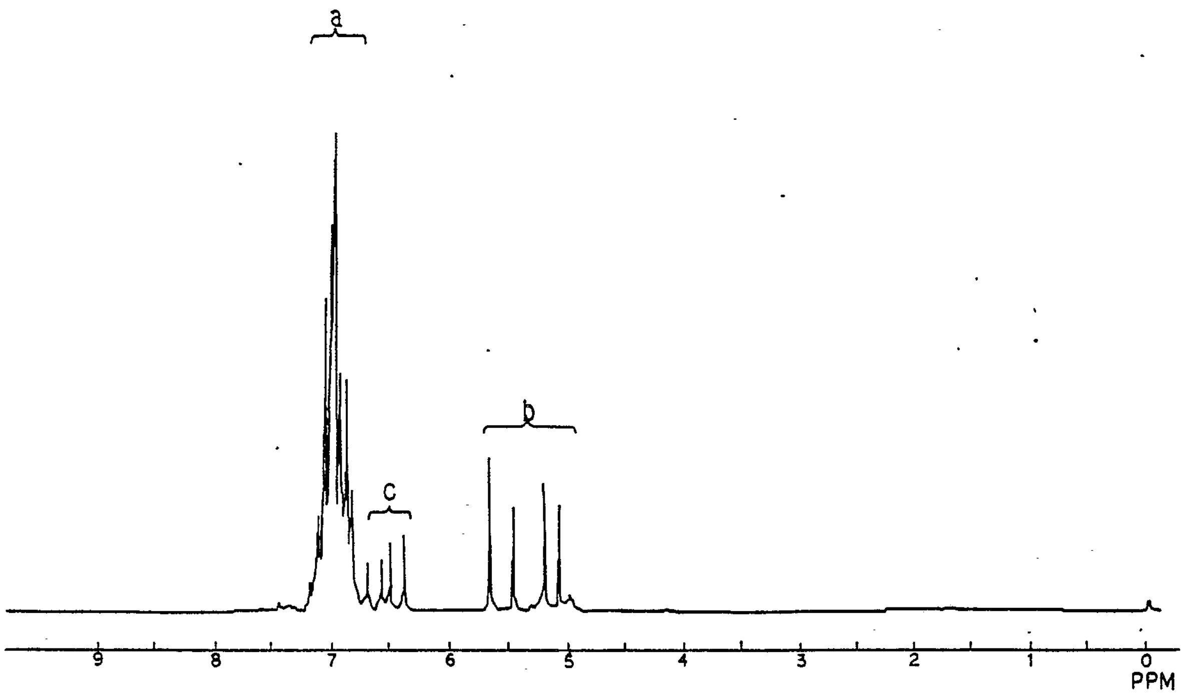


FIG.2

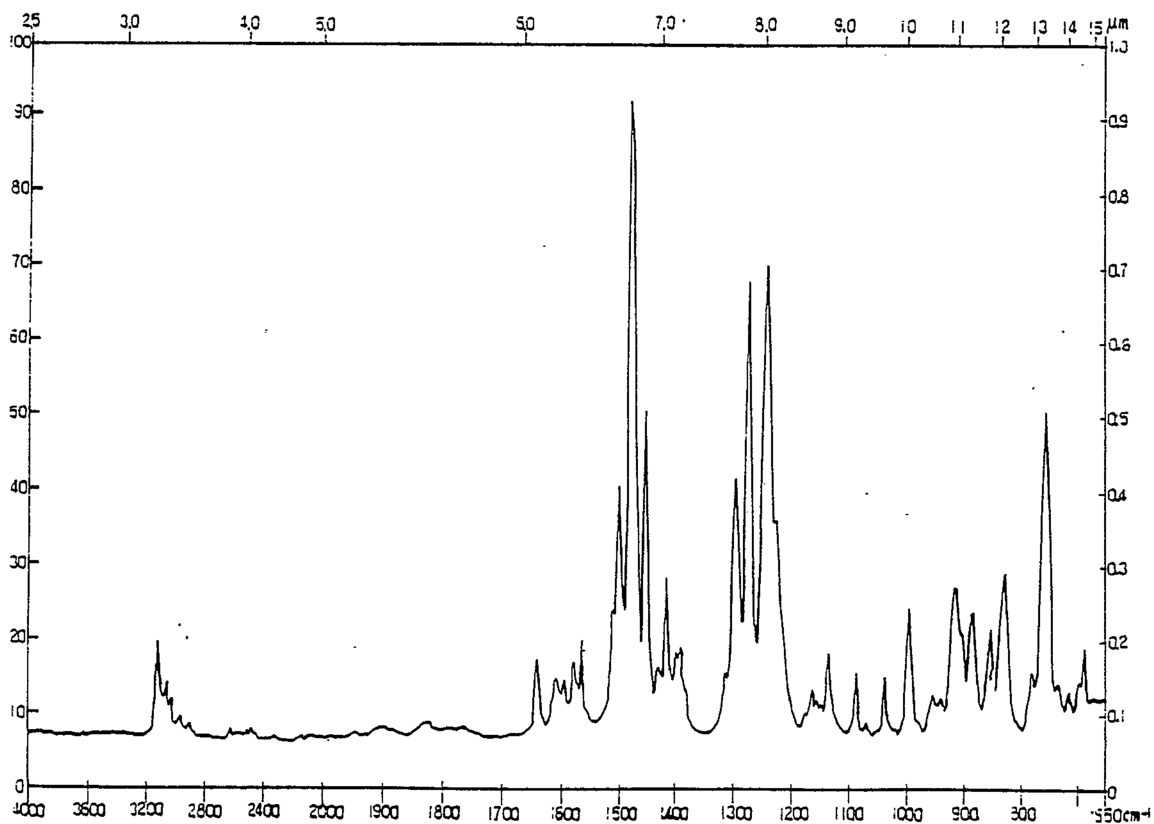

FIG.3

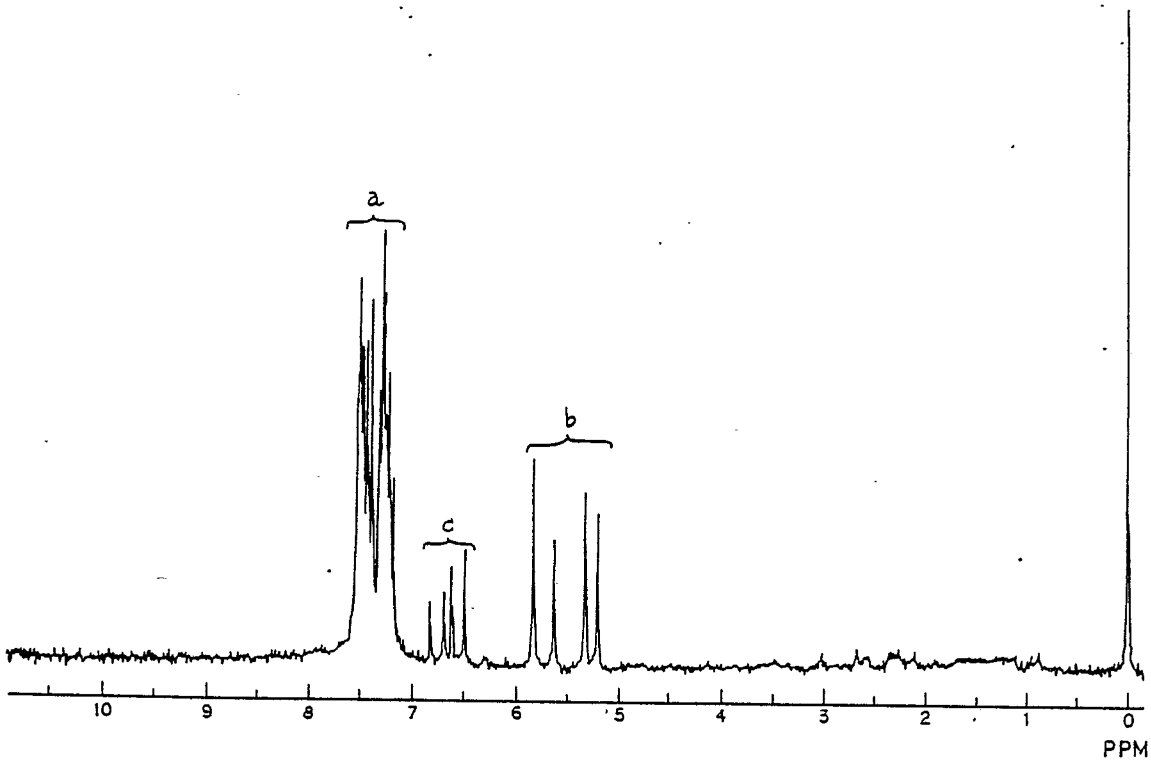
a
b
c
10
9
8
7
6
5
4
3
2
1
0
PPM

FIG.4

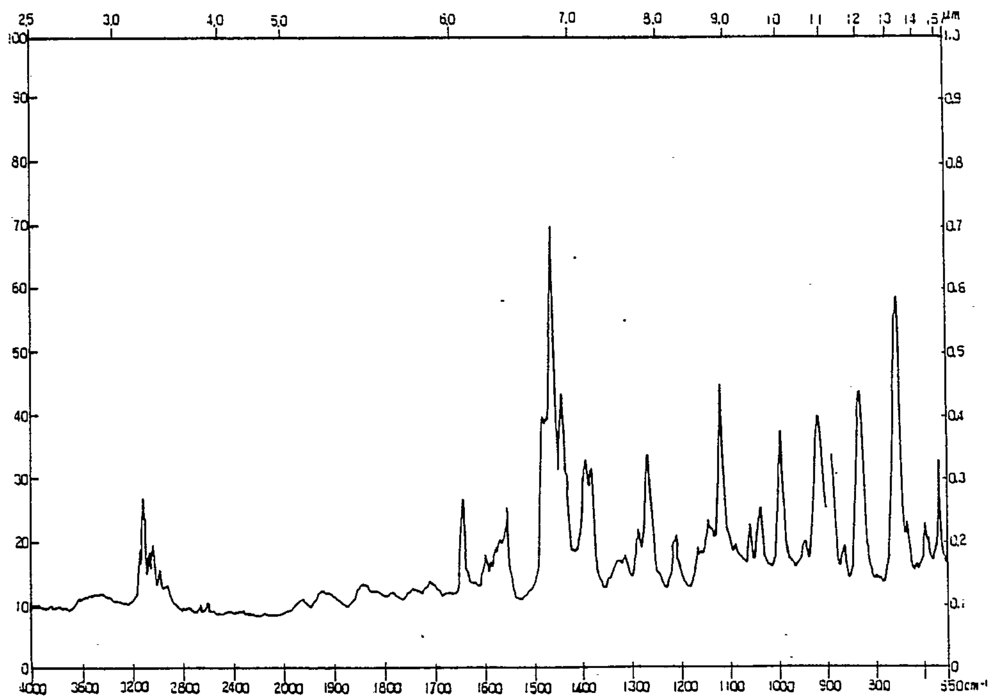
2.5
3.0
4.0
5.0
6.0
7.0
8.0
9.0
10
11
12
13
14
15
μm
100
90
80
70
60
50
40
30
20
10
0
4000
3600
3200
2800
2400
2000
1900
1800
1700
1600
1500
1400
1300
1200
1100
1000
900
800
650cm⁻¹

FIG.5

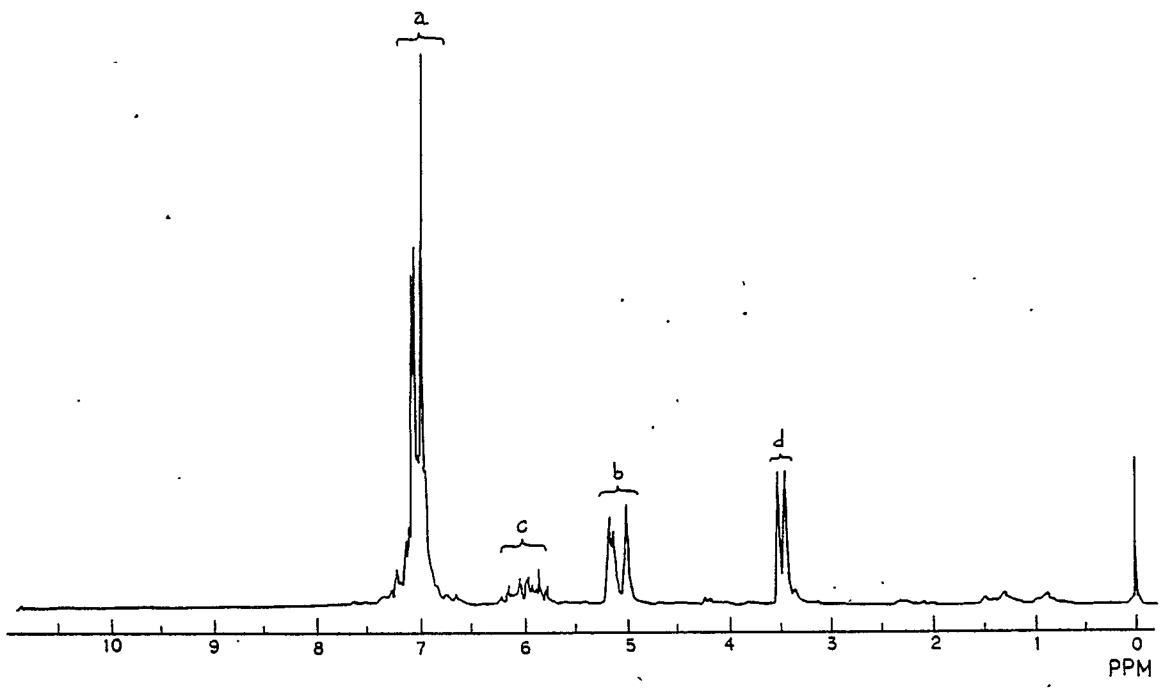


FIG.6

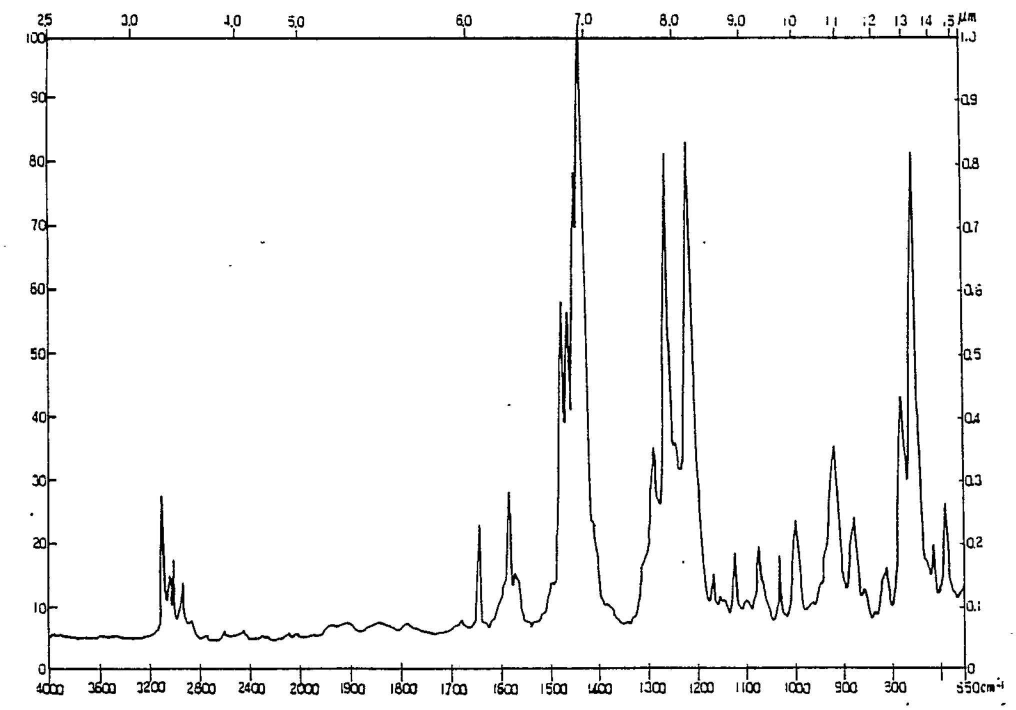

FIG.7

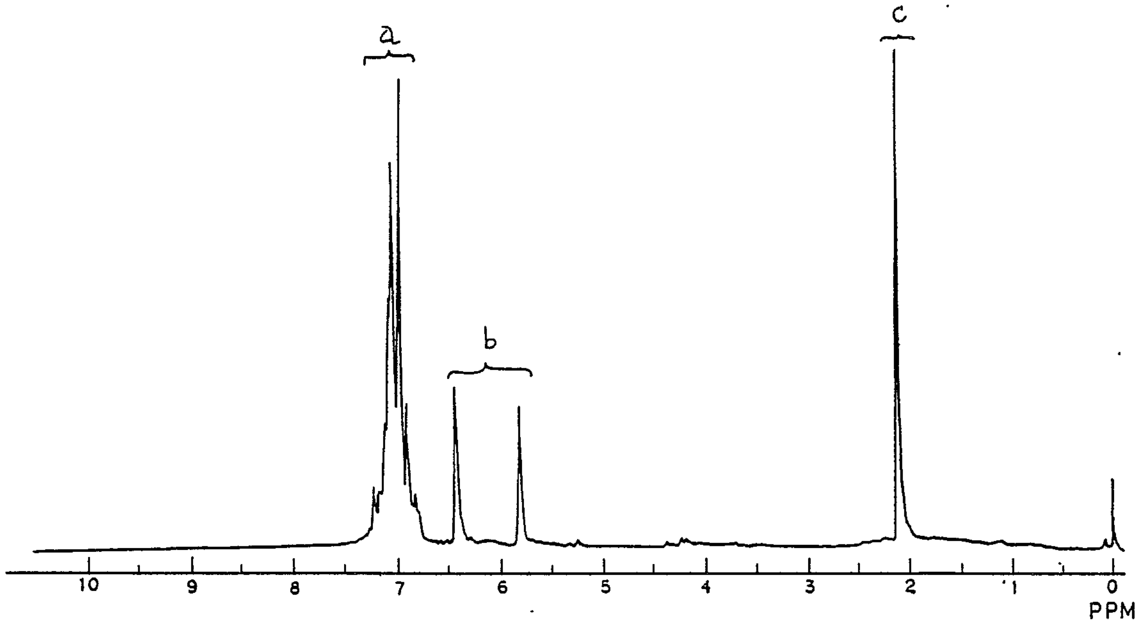
a
b
c
10
9
8
7
6
5
4
3
2
1
0
PPM

FIG.8

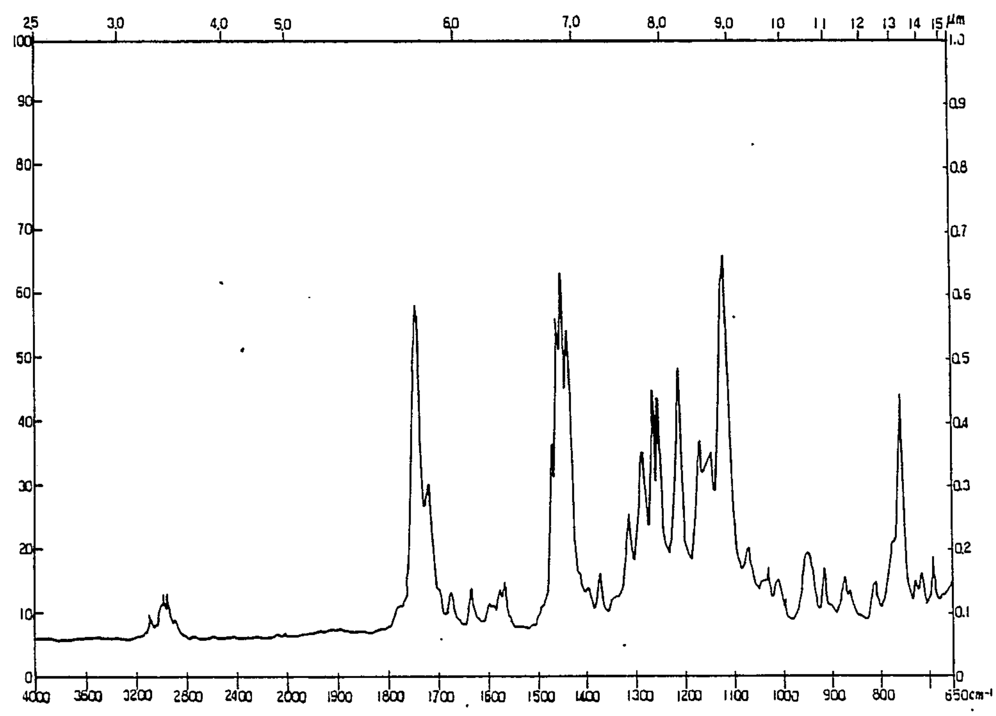
2.5
3.0
4.0
5.0
6.0
7.0
8.0
9.0
10
11
12
13
14
15
μm
100
90
80
70
60
50
40
30
20
10
0
1.0
0.9
0.8
0.7
0.6
0.5
0.4
0.3
0.2
0.1
0
4000
3600
3200
2800
2400
2000
1900
1800
1700
1600
1500
1400
1300
1200
1100
1000
900
800
650cm-1

FIG.9

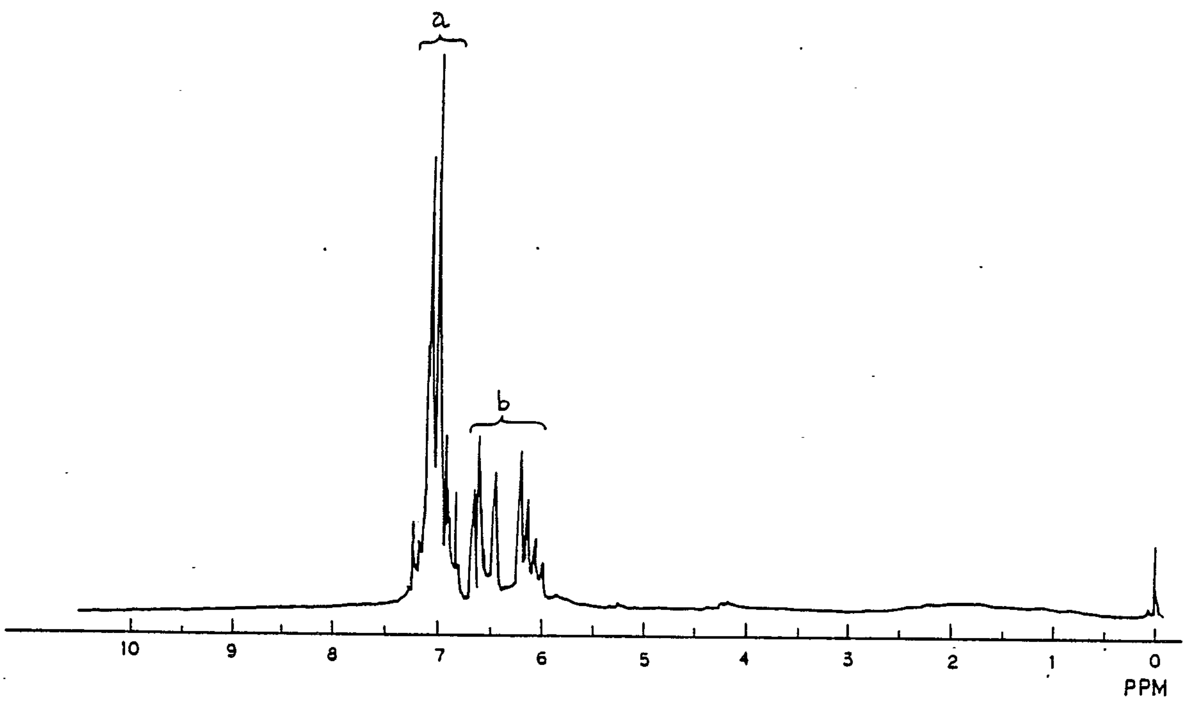
a
b
10
9
8
7
6
5
4
3
2
1
0
PPM

FIG.10

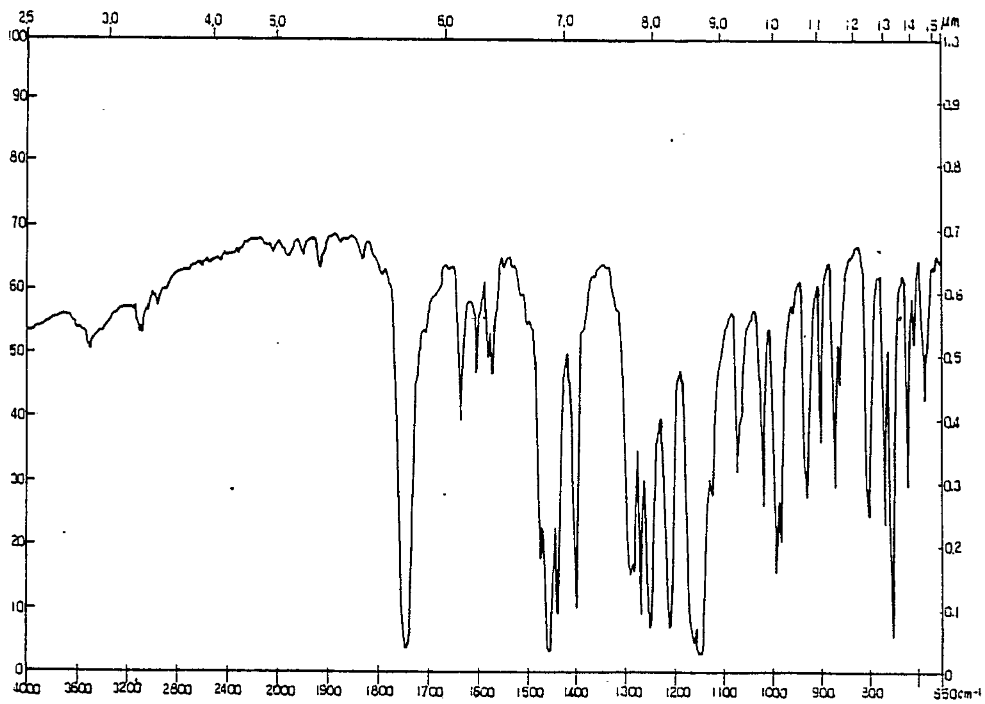
2,5
3,0
4,0
5,0
6,0
7,0
8,0
9,0
10
11
12
13
14
15
μm
100
90
80
70
60
50
40
30
20
10
0
1,0
0,9
0,8
0,7
0,6
0,5
0,4
0,3
0,2
0,1
0
4000
3600
3200
2800
2400
2000
1900
1800
1700
1600
1500
1400
1300
1200
1100
1000
900
800
650cm-1